(19) Europäisches Patentamt
European Patent Office
Office européen des brevets

(11) Veröffentlichungsnummer: **0 126 380**
**A1**

(12) **EUROPÄISCHE PATENTANMELDUNG**

(21) Anmeldenummer: **84105236.8**

(22) Anmeldetag: **09.05.84**

(51) Int. Cl.³: **C 07 D 335/02**, A 01 N 43/18

(30) Priorität: **21.05.83 DE 3318648**

(43) Veröffentlichungstag der Anmeldung: **28.11.84 Patentblatt 84/48**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Gayer, Herbert, Dr., Alfred-Delp-Strasse 4, D-4019 Monheim (DE)**
Erfinder: **Krämer, Wolfgang, Dr., Am Eckbusch 39/45, D-5600 Wuppertal 1 (DE)**
Erfinder: **Brandes, Wilhelm, Dr., Eichendorffstrasse 3, D-5653 Leichlingen (DE)**
Erfinder: **Hänssler, Gerd, Dr., Heymannstrasse 40, D-5090 Leverkusen 1 (DE)**
Erfinder: **Reinecke, Paul, Dr., Lessingstrasse 11, D-5090 Leverkusen 3 (DE)**

(54) **Substituierte Tetrahydrothiopyran-3,5-dion-4-carboxamide.**

(57) Die Erfindung betrifft neue substituierte Tetrahydrothiopyran-3,5-dion-4-carboxamide, ein Verfahren zu ihrer Herstellung und ihre Verwendung als Schädlingsbekämpfungsmittel.
Die neuen substituierten Tetrahydrothiopyran-3,5-dion-4-carboxamide der allgemeinen Formel (I),

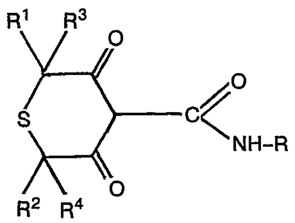

(I)

in welcher R, $R^1$, $R^2$, $R^3$ und $R^4$ die in der Beschreibung gegebene Bedeutung haben, werden aus Tetrahydrothiopyran-3,5-dionen und Isocyanaten hergestellt.
Die Verbindungen sind für den Gebrauch als Schädlingsbekämpfungsmittel, vor allem als Fungizide geeignet.

EP 0 126 380 A1

BAYER AKTIENGESELLSCHAFT 5090 Leverkusen, Bayerwerk

Konzernverwaltung RP
Patentabteilung

Bas/Klu-c
Ia

## Substituierte Tetrahydrothiopyran-3,5-dion-4-carboxamide

Die Erfindung betrifft neue substituierte Tetrahydrothiopyran-3,5-dion-4-carboxamide, ein Verfahren zu ihrer Herstellung und ihre Verwendung als Schädlingsbekämpfungsmittel.

Es ist bereits bekannt, daß einige als Insektizide bekannte Tetrahydrothiopyran-3,5-dion-4-carboxamide, wie beispielsweise das Tetrahydrothiopyran-3,5-dion-4-/N-(3,4-dichlorphenyl)/-carboxamid fungizide Nebenwirkungen besitzen (Vergl. Jap. Pat. 77 46 078 vom 12.4. 1977).

Weiterhin ist eine fungizide und bakterizide Wirkung von unsubstituierten Tetrahydrothiopyran-3,5-dion-4-carboxamiden, wie beispielsweise von Tetrahydrothiopyran-3,5-dion-4-(N-phenyl)-carboxamid, auf dem Pharmasektor bekannt (vgl. US-Patent 3.833.610).

Weiterhin ist bekannt, daß organische Schwefelverbindungen, wie beispielsweise Zink-ethylen-1,2-bis-dithiocarbamat,

fungizide Eigenschaften besitzen (Vergl. R. Wegler, 'Chemie der Pflanzenschutz- und Schädlingsbekämpfungsmittel' Band 2, S. 65, Springer Verlag, Berlin, Heidelberg, New York 1970).

Die Wirkung dieser Verbindungen ist jedoch, insbesondere bei niedrigen Aufwandmengen und -konzentrationen, nicht immer völlig zufriedenstellend.

Es wurden neue substituierte Tetrahydrothiopyran-3,5-dion-4-carboxamide der allgemeinen Formel (I),

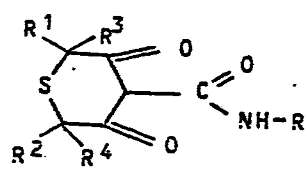
(I)

in welcher

R für Alkyl, für Cycloalkyl, für gegebenenfalls substituiertes Aralkyl oder für gegebenenfalls substituiertes Aryl steht,

$R^1$, $R^2$ und $R^3$ unabhängig voneinander für Wasserstoff oder für Alkyl stehen und

$R^4$ für Alkyl steht, ausgenommen sind die Verbindungen, in denen $R^1$ und $R^3$ für Wasserstoff, $R^2$ und $R^4$ für Methyl und R für Alkyl, Phenyl oder durch Halogen und/oder Methyl substituiertes Phenyl steht, ebenfalls ausgenommen sind Verbindungen, in denen

$R^1$, $R^2$ und $R^3$ für Wasserstoff,
$R^4$ für Methyl und
R für Alkyl, Phenyl oder durch Halogen und/oder Methyl substituiertes Phenyl steht,

gefunden.

Die Verbindungen der Formel (I) liegen im tautomeren Gleichgewicht vor mit Verbindungen der Formeln (Ia) und (Ib):

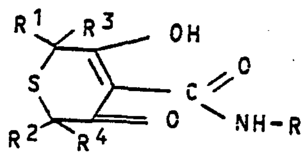 (Ia) 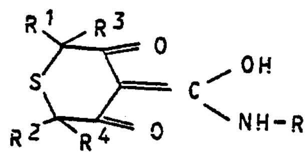 (Ib)

in welcher

R, $R^1$, $R^2$, $R^3$ und $R^4$ die oben angegebene Bedeutung haben.

Besonders die Enolformen (Ia) und (Ib) werden durch starke intramolekulare Wasserstoffbrückenbindungen stabilisiert.

Weiterhin können die Verbindungen der Formel (I) als geometrische Isomere oder Isomerengemische unterschiedlicher Zusammensetzung vorliegen. Sowohl die reinen Isomeren als auch die Isomerengemische, ebenso wie die verschiedenen tautomeren Strukturen werden erfindungsgemäß beansprucht.

Weiterhin wurde gefunden, daß man die neuen substituierten Tetrahydrothiopyran-3,5-dion-4-carboxamide der allgemeinen Formel (I),

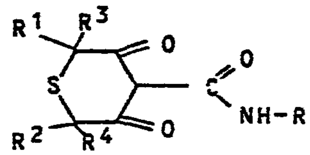

(I)

in welcher

R für Alkyl, für Cycloalkyl, für gegebenenfalls subsitutiertes Aralkyl oder für gegebenenfalls substituiertes Aryl steht,

$R^1$, $R^2$ und $R^3$ unabhängig voneinander für Wasserstoff oder für Alkyl stehen und

$R^4$ für Alkyl steht, ausgenommen die Verbindungen, in denen

$R^1$ und $R^3$ für Wasserstoff,
$R^2$ und $R^4$ für Methyl und

R für Alkyl, Phenyl oder durch Halogen und/oder Methyl substituiertes Phenyl steht oder

$R^1$, $R^2$ und $R^3$ für Wasserstoff,

$R^4$ für Methyl und

R für Alkyl, Phenyl oder durch Halogen und/oder Methyl substituiertes Phenyl stehen,

erhält, wenn man Tetrahydrothiopyran-3,5-dione der allgemeinen Formel (II)

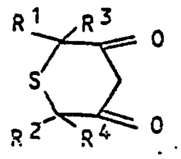

(II)

in welcher

$R^1$, $R^2$, $R^3$ und $R^4$ die oben angegebene Bedeutung haben,

mit Isocyanaten der allgemeinen Formel (III),

$$R - N = C = O \qquad (III)$$

in welcher

R die oben angegebene Bedeutung hat,

gegebenenfalls in Gegenwart eines Verdünnungsmittels und gegebenenfalls in Gegenwart einer Base zur Reaktion bringt.

Die neuen substituierten Tetrahydrothiopyran-3,5-dion-4-carboxamide der allgemeinen Formel (I) besitzen starke fungizide Eigenschaften. Dabei zeigen die erfindungsgemäßen Verbindungen der Formel (I) überraschenderweise eine bessere fungizide Wirksamkeit als die aus dem Stand der Technik bekannten Verbindungen Tetrahydrothiopyran-3,5-dion-4-/N̄-(3,4-dichlorphenyl)7-carboxamid bzw. Zinkethylen-1,2-bis-dithiocarbamat, welches chemisch bzw. wirkungsmäßig naheliegende Verbindungen sind.

Die erfindungsgemäßen Stoffe stellen somit eine Bereicherung der Technik dar.

Die erfindungsgemäßen substituierten Tetrahydrothiopyran-3,5-dion-4-carboxamide sind durch die Formel (I) allgemein definiert.

Bevorzugt sind Verbindungen der Formel (I), bei denen

R für geradkettiges oder verzweigtes Alkyl mit bis zu 8 Kohlenstoffatomen, für Cycloalkyl mit 3 bis 7 Kohlenstoffatomen, für gegebenenfalls einfach oder mehrfach, gleich oder verschieden substituiertes Aralkyl mit 1 oder 2 Kohlenstoffatomen im Alkylteil und 6 bis 10 Kohlenstoffatomen im Arylteil, sowie für gegebenenfalls einfach oder mehrfach, gleich oder verschieden substituiertes Aryl mit 6 bis 10 Kohlenstoffatomen steht, wobei als Arylsubstituenten jeweils infrage kommen: Halogen, Cyano, Nitro, geradkettiges oder verzweigtes Alkyl, Alkoxy und Alkylthio mit jeweils bis zu 4 Kohlenstoffatomen, Halogenalkyl, Halogenalkoxy und Halogenalkylthio mit jeweils 1 oder 2 Kohlenstoffatomen und bis zu 5 gleichen oder verschiedenen Halogenatomen, gegebenenfalls durch Halogen oder

niederes Alkyl einfach oder mehrfach, gleich oder verschieden substituiertes Dioxyalkylen mit 1 oder 2 Kohlenstoffatomen im Alkylenrest, gegebenenfalls einfach oder mehrfach, gleich oder verschieden durch Halogen, niederes Alkyl und niederes Halogenalkyl substituiertes Phenoxy sowie Alkoxycarbonylalkyl und Alkoxycarbonylalkenyl mit jeweils 1 oder 2 Kohlenstoffatomen im Alkoxyteil und jeweils bis zu 2 Kohlenstoffatomen im Alkyl- bzw. Alkenylteil,

$R^1$, $R^2$ und $R^3$ unabhängig voneinander für Wasserstoff oder für geradkettiges oder verzweigtes Alkyl mit bis zu 6 Kohlenstoffatomen stehen und

$R^4$ für geradkettiges oder verzweigtes Alkyl mit 1 bis 6 Kohlenstoffatomen steht, ausgenommen die Verbindungen in denen

$R^1$ und $R^3$ für Wasserstoff,

$R^2$ und $R^4$ für Methyl und

R für Alkyl, Phenyl oder durch Halogen und/oder Methyl substituiertes Phenyl steht oder

$R^1$, $R^2$ und $R^3$ für Wasserstoff,

$R^4$ für Methyl und

R für Alkyl, Phenyl oder durch Halogen und/oder Methyl substituiertes Phenyl stehen.

Ganz besonders bevorzugt sind Verbindungen der Formel (I), bei denen

R für geradkettiges oder verzweigtes Alkyl mit bis zu 6 Kohlenstoffatomen, für Cyclopentyl, für Cyclohexyl oder für gegebenenfalls ein- bis dreifach, gleich oder verschieden substituiertes Phenyl, Benzyl oder Naphthyl steht, wobei als Substituenten jeweils infrage kommen: Fluor, Chlor, Brom, Nitro, Cyano, Methyl, Ethyl, n- und i-Propyl, n-, i-, s- und t-Butyl, Methoxy, Ethoxy, Methylthio, Trifluormethyl, Trifluormethoxy, Trifluormethylthio, Dioxymethylen, Dioxyethylen, Dioxytrifluorchlorethylen, gegebenenfalls ein- bis dreifach, gleich oder verschieden durch Methyl, Chlor oder Trifluormethyl substituiertes Phenoxy, 2-Methoxycarbonylvinyl und 2-Ethoxycarbonylvinyl,

$R^1$, $R^2$ und $R^3$ unabhängig voneinander für Wasserstoff, Methyl, Ethyl, n- und i-Propyl sowie für n-, i-, s- und t-Butyl stehen und

$R^4$ für Methyl, Ethyl, n- und i-Propyl, n-, i-, s- und t-Butyl steht, ausgenommen die Verbindungen, in denen

$R^1$ und $R^3$ für Wasserstoff,

$R^2$ und $R^4$ für Methyl und

R für Alkyl, Phenyl oder durch Halogen und/oder Methyl substituiertes Phenyl stehen oder in denen

$R^1$, $R^2$ und $R^3$ für Wasserstoff,

$R^4$ für Methyl und

R für Alkyl, Phenyl oder durch Halogen und/oder Methyl substituiertes Phenyl stehen.

Im einzelnen seien außer den bei den Herstellungsbeispielen genannten Verbindungen die folgenden Verbindungen der allgemeinen Formel (I) genannt:

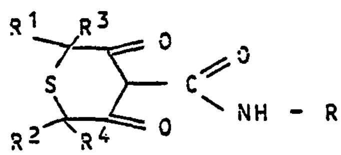 (I)

| R | $R^1$ | $R^2$ | $R^3$ | $R^4$ |
|---|---|---|---|---|
| 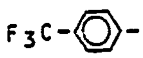 | H | H | H | $C_2H_5$ |
| 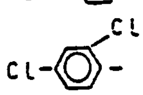 | H | H | H | $C_2H_5$ |
| 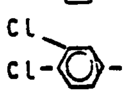 | H | H | H | $C_2H_5$ |
| 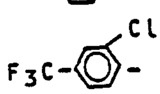 | H | H | H | $C_2H_5$ |
| 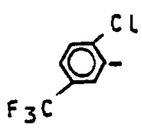 | H | H | H | $C_2H_5$ |
| 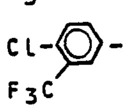 | H | H | H | $C_2H_5$ |
| 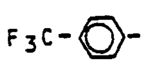 | H | H | H | $CH_3-(CH_2)_2-$ |

| R | $R^1$ | $R^2$ | $R^3$ | $R^4$ |
|---|---|---|---|---|
| 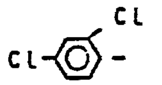 | H | H | H | $CH_3-(CH_2)_2-$ |
| 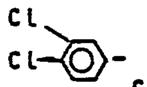 | H | H | H | $CH_3-(CH_2)_2-$ |
| 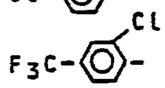 | H | H | H | $CH_3-(CH_2)_2-$ |
| 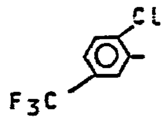 | H | H | H | $CH_3-(CH_2)_2-$ |
| 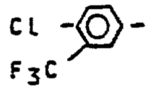 | H | H | H | $CH_3-(CH_2)_2-$ |
| 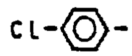 | H | H | H | $CH_3(CH_2)_3-$ |
| 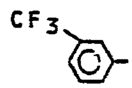 | H | H | H | $CH_3(CH_2)_3-$ |
| 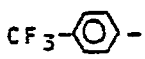 | H | H | H | $CH_3(CH_2)_3-$ |
| 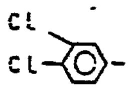 | H | H | H | $CH_3(CH_2)_3-$ |
| 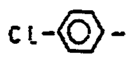 | H | H | H | 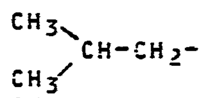 |
| 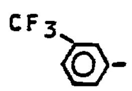 | H | H | H | 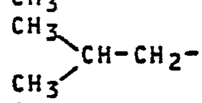 |
| 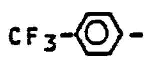 | H | H | H | 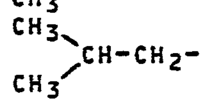 |
| 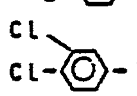 | H | H | H | 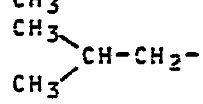 |
| 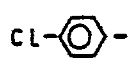 | H | $CH_3$ | H | $C_2H_5$ |
| 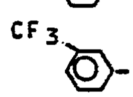 | H | $CH_3$ | H | $C_2H_5$ |

| R | $R^1$ | $R^2$ | $R^3$ | $R^4$ |
|---|---|---|---|---|
| 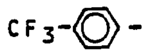 | H | $CH_3$ | H | $C_2H_5$ |
| 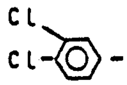 | H | $CH_3$ | H | $C_2H_5$ |
| 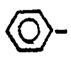 | H | H | H | $C_2H_5$ |
| 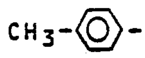 | H | H | H | $C_2H_5$ |
| 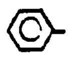 | H | H | H | $CH_3(CH_2)_2-$ |
| 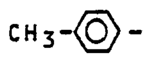 | H | H | H | $CH_3(CH_2)_2-$ |
| 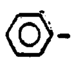 | H | H | H | $CH_3(CH_2)_3-$ |
| 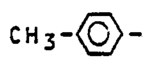 | H | H | H | $CH_3(CH_2)_3-$ |
| 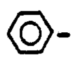 | H | H | H | 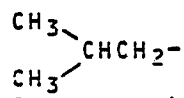 |
| 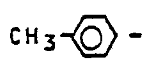 | H | H | H | 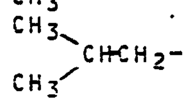 |
| 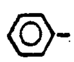 | H | $CH_3$ | H | $C_2H_5$ |
| 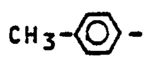 | H | $CH_3$ | H | $C_2H_5$ |

Verwendet man beispielsweise 2,6-Dimethyltetrahydrothiopyran-3,5-dion und Phenylisocyanat als Ausgangsstoffe, so kann der Reaktionsablauf des erfindungsgemäßen Verfahrens durch das folgende Formelschema wiedergegeben werden:

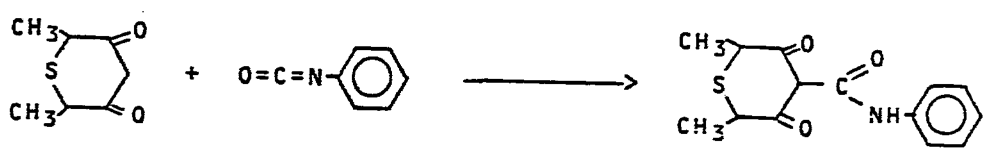

Die bei der Durchführung des erfindungsgemäßen Verfahrens als Ausgangsstoffe benötigten Tetrahydrothiopyran-3,5-dione sind durch die Formel (II) allgemein definiert. Die Tetrahydrothiopyran-3,5-dione der Formel (II) sind teilweise bekannt /vergl. J. Amer. Chem. Soc. 97, 2718-2742 (1975)7. Die nicht bekannten Vertreter lassen sich in analoger Weise herstellen, indem man α-Halogenketone der Formel (IV),

$$CH_3-CO-\overset{R^4}{\underset{R^2}{C}}-Hal \qquad (IV)$$

in welcher

$R^2$ und $R^4$ die oben angegebene Bedeutung haben

und

Hal für Chlor oder Brom steht,

mit α-Mercaptocarbonsäureestern der Formel (V),

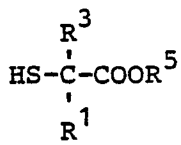

(V)

in welcher

$R^1$ und $R^3$ die oben angegebene Bedeutung haben und

$R^5$ für geradkettiges oder verzweigtes Alkyl mit bis zu 4 Kohlenstoffatomen steht,

gegebenenfalls in Gegenwart eines Verdünnungsmittels, wie beispielsweise Methanol, und gegebenenfalls in Gegenwart einer Base, wie beispielsweise Natriummethylat, bei Temperaturen zwischen -20°C und +50°C zu den Estern der Formel (VI) umsetzt,

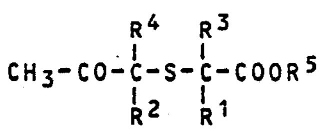

(VI)

in welcher

$R^1$, $R^2$, $R^3$, $R^4$ und $R^5$ die oben angegebene Bedeutung haben,

und diese in einer 2. Stufe gegebenenfalls in Gegenwart eines Verdünnungsmittels, wie beispielsweise Toluol, und gegebenenfalls in Gegenwart einer Base, wie beispielsweise Natriummethylat, zu den Tetrahydrothiopyran-3,5-dionen der Formel (II) cyclisiert.

Die weiterhin zur Durchführung des erfindungsgemäßen Verfahrens als Ausgangsstoffe benötigten Isocyanate sind durch die Formel (III) allgemein definiert.

Isocyanate der Formel (III), α-Halogenketone der Formel (IV) und α-Mercaptocarbonsäureester der Formel (V) sind allgemein bekannte Verbindungen der organischen Chemie.

Als Verdünnungsmittel kommen für das erfindungsgemäße Verfahren inerte organische Lösungsmittel infrage. Hierzu gehören vorzugsweise aliphatische und aromatische gegebenenfalls chlorierte Kohlenwasserstoffe, wie Benzin, Benzol, Toluol, Xylol, Methylenchlorid, Chloroform, Tetrachlorkohlenstoff, Chlorbenzol und Dichlorbenzol, Ether, wie Diethylether, Diisopropylether, Dioxan und Tetrahydrofuran, Ketone, wie Aceton, Butanon und Methylisopropylketon, Nitrile, wie Acetonitril und Propionitril, sowie die hochpolaren Lösungsmittel Dimethylsulfoxid und Hexamethylphosphorsäuretriamid.

Die erfindungsgemäße Umsetzung kann gegebenenfalls in Gegenwart einer Base durchgeführt werden. Bevorzugt verwendet man tertiäre organische Stickstoffbasen wie Triethylamin, N,N-Dimethylbenzylamin, N,N-Dimethylanilin, Diazabicyclononen (DBN) oder Diazabicycloundecen (DBU).

Die Reaktionstemperaturen können bei dem erfindungsgemäßen Verfahren in einem größeren Bereich variiert werden. Im allgemeinen arbeitet man bei Temperaturen zwischen -10°C und + 100°C, vorzugsweise bei Temperaturen zwischen +10°C und +80°C.

Zur Durchführung des erfindungsgemäßen Verfahrens setzt man pro Mol Tetrahydrothiopyran-3,5-dion der Formel (II) im allgemeinen 1,0 bis 1,2 Mol, vorzugsweise äquimolare Mengen, an Isocyanat der Formel (III) und gegebenenfalls 1,0 bis 1,2 Mol, vorzugsweise äquimolare Mengen, an Base ein. Nach ein bis zwei Stunden Reaktionszeit bei der erforderlichen Temperatur läßt man die Mischung auf Raumtemperatur kommen und entfernt die Base durch Extraktion mit Wasser und das Verdünnungsmittel durch Destillation im Vakuum.

Zur Isolierung und Reinigung der Verbindungen der Formel (I) chromatographiert man den öligen Destillationsrückstand über eine Kieselgelsäule mit einem Laufmittelgemisch aus Ether, Petrolether und Essigsäure. Die derart gereinigten Verbindungen der Formel (I) liegen entweder als Oele oder als kristalline Feststoffe vor, die sich aus Ethanol umkristallisieren lassen. Zur Charakterisierung dienen der Schmelzpunkt oder das $^{1}H$-NMR-Spektrum.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten wie beispielsweise gegen den Erreger des Schneeschimmels an Roggen (Fusarium nivale) oder gegen den Erreger des Braunrostes am Weizen (Puccinia recondita), zur Bekämpfung von Obst- und Gemüsekrankheiten, wie beispielsweise gegen den Erreger des Apfelschorfs (Venturia inaequalis) oder gegen den Erreger der Tomatenbraunfäule (Phytophtora infestans), oder zur Bekämpfung von Reiskrankheiten, wie beispielsweise gegen den Erreger der Reisfleckenkrankheit (Pyricularia oryzae), angewendet werden. Weiterhin ist die gute Wirkung gegen Cochliobolus sativus und Pyrenophora teres am Getreide zu erwähnen.

Dabei zeigen die erfindungsgemäßen Wirkstoffe sowohl protektive als auch systemische Wirksamkeit und können auch als Saatgutbeizmittel erfolgreich angewendet werden.

Daneben besitzen die erfindungsgemäßen Wirkstoffe auch eine insektizide und akarizide Wirksamkeit.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quarz, Attapulgit, Montmorrillonit oder Diateomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen,

Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrachsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-% vorzugsweise von 0,001 bis 0,02 Gew.-%, am Wirkungsort erforderlich.

## Herstellungsbeispiele

### Beispiel 1

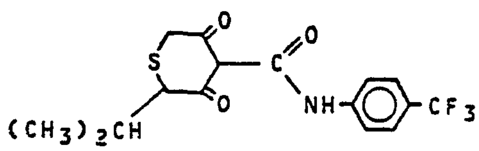

Zu 172 g (1 Mol) 2-Isopropyl-tetrahydrothiopyran-3,5-dion in 1 l Tetrahydrofuran tropft man bei 25°C unter Rühren zunächst 124 g (1 Mol) Diazabicycloundecen (DBU) und danach 188 g (1 Mol) 4-Trifluormethylphenylisocyanat. Nach beendeter Zugabe rührt man eine Stunde bei Raumtemperatur, wäscht mehrfach mit wässriger 2 N Salzsäure und entfernt dann das Lösungsmittel im Vakuum. Der ölige Rückstand wird durch Chromatographie an Kieselgel (Laufmittel: Ether/Petrolether/Eisessig im Verhältnis 10:10:0,3) gereinigt. Man erhält 190 g (53 % der Theorie an 2-Isopropyltetrahydrothiopyran-3,5-dion-4-/N-(4-trifluormethylphenyl)/-carboxamid vom Schmelzpunkt 121°C.

Herstellung der Ausgangsverbindung:

a) 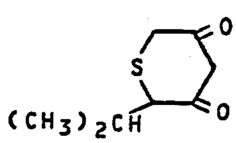

Zu einer Suspension von 108 g (2 Mol) frisch hergestelltem Natriummethylat in 2 l absolutem Toluol tropft man innerhalb einer Stunde bei 0°C unter kräftigem Rühren 204 g (1 Mol) 3-Methoxycarbonylmethylthio-4-methylpentan-2-on. Man rührt weitere 30 Minuten bei 0°C nach, gießt dann die Reaktionsmischung in ein Gemisch aus 200 ml konzentrierter Salzsäure und 800 ml Eiswasser, trennt die organische Phase ab und extrahiert die wässrige Phase dreimal mit Dichlormethan. Die vereinigten organischen Phasen werden im Vakuum auf 500 ml eingeengt und mit 500 ml 4N eiskalter Natronlauge extrahiert. Die wässrigen Extrakte werden sofort mit insgesamt 400 ml eiskalter Salzsäure angesäuert und dreimal mit je 200 ml Dichlormethan extrahiert. Die vereinigten Dichlormethanphasen werden über Natriumsulfat getrocknet und im Vakuum vom Lösungsmittel befreit. Der zurückbleibende Feststoff wird aus Ligroin/Ethanol umkristallisiert. Man erhält 150 g (87 % der Theorie) an 2-Isopropyltetrahydrothiopyran-3,5-dion vom Schmelzpunkt 71°C.

b)

$CH_3-CO-CH-S-CH_2-COOCH_3$
$\qquad\qquad\quad | $
$\qquad\qquad CH(CH_3)_2$

Zu einer Lösung von 54 g (1 Mol) Natriummethylat in 1 l Methanol tropft man bei 0°C unter Rühren zunächst 106 g (1 Mol) Mercaptoessigsäuremethylester und danach 165 g (1 Mol) 3-Brom-3-methyl-butan-2-on. Nach beendeter

Zugabe kocht man das Reaktionsgemisch 30 Minuten am Rückfluß, filtriert nach dem Erkalten abgeschiedenes Natriumbromid ab und entfernt das Lösungsmittel im Vakuum. Der Rückstand wird zwischen einer Mischung aus 500 ml Dichlormethan und 500 ml Wasser verteilt, die organische Phase wird abgetrennt, über Natriumsulfat getrocknet und im Vakuum vom Lösungsmittel befreit. Der Rückstand wird im Hochvakuum destilliert. Man erhält 122,5 g (60 % der Theorie) an 3-Methoxycarbonylmethylthio-4-methyl-pentan-2-on vom Siedepunkt 80°C bei 0,4 mbar.

In entsprechender Weise und gemäß den allgemeinen Herstellungsangaben erhält man die folgenden Verbindungen der allgemeinen Formel (I):

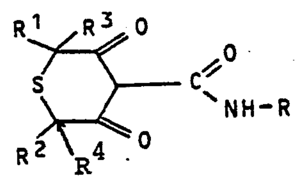

(I)

| Bsp. Nr. | R | $R^1$ | $R^2$ | $R^3$ | $R^4$ | physikalische Eigenschaften |
|---|---|---|---|---|---|---|
| 2 | 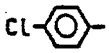 | H | H | H | $C_2H_5$ | Fp: 68°C |
| 3 | 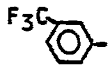 | H | H | H | $C_2H_5$ | $^{1}$H-NMR:* 0,8-1,4(3);1,6-2,3(2); 3,0-3,7(3);7,1-7,0(4) |
| 4 | 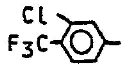 | H | H | H | $C_2H_5$ | Fp: 95°C |
| 5 | 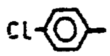 | H | H | H | $CH_3(CH_2)_2-$ | Fp:81°C |
| 6 | 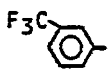 | H | H | H | $CH_3(CH_2)_2-$ | $^{1}$H-NMR: 0,7-1,1(3);1,1-2,2(4); 3,1-3,6(3);7,1-8,0(4) |
| 7 | 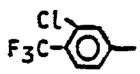 | H | H | H | $CH_3(CH_2)_2-$ | Fp:82°C |
| 8 | 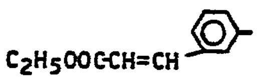 | H | H | H | $CH_3(CH_2)_2-$ | Fp:62-72°C |
| 9 | 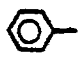 | H | H | H | $(CH_3)_2CH-$ | Fp:68-69°C |
| 10 | 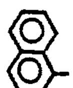 | H | H | H | $(CH_3)_2CH-$ | Fp:150°C |
| 11 | 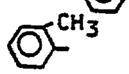 | H | H | H | $(CH_3)_2CH-$ | Fp:95-97°C |
| 12 | 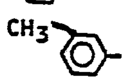 | H | H | H | $(CH_3)_2CH-$ | $^{1}$H-NMR: 0,7-1,3(6);2,3s(3); 2,0-2,5(1);2,8-3,8(3); 6,7-7,4(4) |
| 13 | 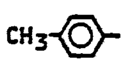 | H | H | H | $(CH_3)_2CH-$ | Fp:73°C |

| Bsp. Nr. | R | $R^1$ | $R^2$ | $R^3$ | $R^4$ | physikal. Eigenschaften |
|---|---|---|---|---|---|---|
| 14 | 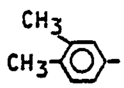 | H | H | H | $(CH_3)_2CH-$ | Fp:77-79°C |
| 15 | 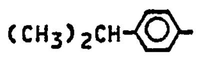 | H | H | H | $(CH_3)_2CH-$ | $^1H$-NMR: 0,7-1,4(12);2,0-3,8(5); 7,0-7,5(4), |
| 16 | 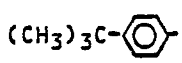 | H | H | H | $(CH_3)_2CH-$ | $^1H$-NMR: 1,35s(9); 0,7-1,4(6);2,1-2,8(1); 3,0-3,9(3); 7,4s(4) |
| 17 | 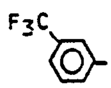 | H | H | H | $(CH_3)_2CH-$ | Fp:62-64°C |
| 18 | 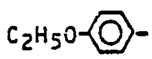 | H | H | H | $(CH_3)_2CH-$ | Fp:101°C |
| 19 | 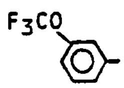 | H | H | H | $(CH_3)_2CH-$ | Fp:61°C |
| 20 | 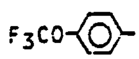 | H | H | H | $(CH_3)_2CH-$ | Fp:65-66°C |
| 21 | 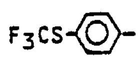 | H | H | H | $(CH_3)_2CH-$ | Fp:70°C |
| 22 | 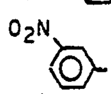 | H | H | H | $(CH_3)_2CH-$ | Fp:75-80°C |
| 23 | 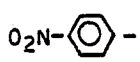 | H | H | H | $(CH_3)_2CH-$ | Fp:124°C |
| 24 | 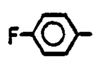 | H | H | H | $(CH_3)_2CH-$ | Fp:60-62°C |
| 25 | 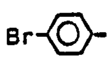 | H | H | H | $(CH_3)_2CH-$ | Fp:118-119°C |
| 26 | 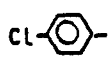 | H | H | H | $(CH_3)_2CH-$ | Fp:111-115°C |
| 27 | 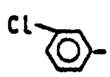 | H | H | H | $(CH_3)_2CH-$ | $^1H$-NMR:1,9-2,2(6); 2,1-2,7(1);2,9-3,9(3); 6,9-7,5(3);7,6-7,5s(1) |
| 28 | 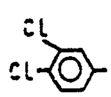 | H | H | H | $(CH_3)_2CH-$ | Fp:89-94°C |

| Bsp. Nr. | R | $R^1$ | $R^2$ | $R^3$ | $R^4$ | physikal. Eigenschaften |
|---|---|---|---|---|---|---|
| 29 | 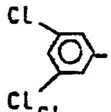 | H | H | H | $(CH_3)_2CH-$ | Fp:103°C |
| 30 | 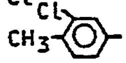 | H | H | H | $(CH_3)_2CH-$ | Fp:72-75°C |
| 31 | 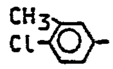 | H | H | H | $(CH_3)_2CH-$ | Fp:101°C |
| 32 | 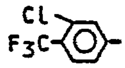 | H | H | H | $(CH_3)_2CH-$ | Fp:111°C |
| 33 | 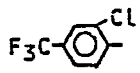 | H | H | H | $(CH_3)_2CH-$ | Fp:135°C |
| 34 | 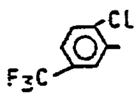 | H | H | H | $(CH_3)_2CH-$ | Fp:171-173°C |
| 35 | 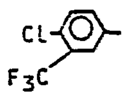 | H | H | H | $(CH_3)_2CH-$ | Fp:98°C |
| 36 | 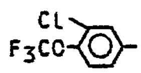 | H | H | H | $(CH_3)_2CH-$ | Fp:55-56°C |
| 37 | 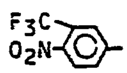 | H | H | H | $(CH_3)_2CH-$ | $^1$H-NMR:0,9-1,4(6); 2,0-2,9(1);3,1-3,9(3); 7,9-8,3(3) |
| 38 | 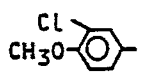 | H | H | H | $(CH_3)_2CH-$ | Fp:99-102°C |
| 39 | 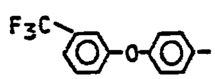 | H | H | H | $(CH_3)_2CH-$ | $^1$H-NMR: 0,7-1,2(6); 2,0-2,6(1);2,7-3,7(2); 6,7-7,7(8) |
| 40 | 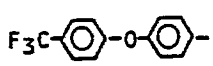 | H | H | H | $(CH_3)_2CH-$ | $^1$H-NMR:0,8-1,3(6); 2,0-2,6(1);2,7-3,7(3); 6,7-7,7(8) |
| 41 | 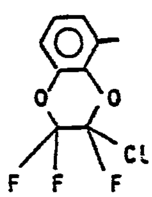 | H | H | H | $(CH_3)_2CH-$ | $^1$H-NMR:0,8-1,3(6); 2,1-2,6(1);2,9-3,6(2); 6,9-7,3(2);7,4-7,6(1) |

| Bsp. Nr. | R | $R^1$ | $R^2$ | $R^3$ | $R^4$ | physikal. Eigenschaften |
|---|---|---|---|---|---|---|
| 42 | $CH_3$ | H | H | $CH_3$ | $CH_3$ | $^1$H-NMR:1,2-1,8(6); 2,95 d (3);3,2-4,1(2) |
| 43 | $(CH_3)_2CH-$ | H | H | $CH_3$ | $CH_3$ | $^1$H-NMR: 1,0-1,8(12); 3,3-4,4(3) |
| 44 | $CH_3(CH_2)_3-$ | H | H | $CH_3$ | $CH_3$ | $^1$H-NMR:0,8-1,15(3); 1,15-1,8(10);3,1-4,0(4) |
| 45 | $(CH_3)_2CH-CH_2-$ | H | H | $CH_3$ | $CH_3$ | $^1$H-NMR:0,8-1,1(6); 1,2-1,9(7);2,8-4,0(4) |
| 46 | $(CH_3)_3C-$ | H | H | $CH_3$ | $CH_3$ | $^1$H-NMR:1,4s(9);1,2-1,8 (6); 3,2-4,0(2) |
| 47 | 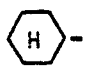 | H | H | $CH_3$ | $CH_3$ | $^1$H-NMR:1,0-2,2(16); 32,-4,0(3) |
| 48 | 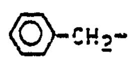 | H | H | $CH_3$ | $CH_3$ | $^1$H-NMR:1,2-1,9(6); 3,3-4,1(2); 4,6d(2); 7,4s(5) |
| 49 | 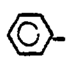 | H | H | $CH_3$ | $CH_3$ | Fp:155°C |
| 50 | 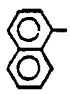 | H | H | $CH_3$ | $CH_3$ | Fp:87-94°C |
| 51 | 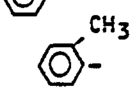 | H | H | $CH_3$ | $CH_3$ | Fp:154°C |
| 52 | 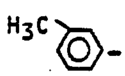 | H | H | $CH_3$ | $CH_3$ | Fp:72-74°C |
| 53 | 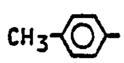 | H | H | $CH_3$ | $CH_3$ | Fp:70°C |
| 54 | 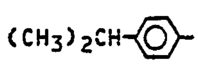 | H | H | $CH_3$ | $CH_3$ | $^1$H-NMR:1-1,8(12); 2,5-3,1(1);3,4-3,9(2); 7,0-7,6(4) |
| 55 | 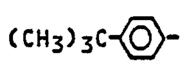 | H | H | $CH_3$ | $CH_3$ | Fp:90-92°C |
| 56 | 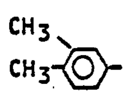 | H | H | $CH_3$ | $CH_3$ | $^1$H-NMR:1,0-1,8; 2,2s(6); 3,2-3,9(2); 6,8-7,5(3) |
| 57 | 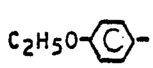 | H | H | $CH_3$ | $CH_3$ | Fp:114-117°C |

| Bsp. Nr: | R | $R^1$ | $R^2$ | $R^3$ | $R^4$ | physikal. Eigenschaften |
|---|---|---|---|---|---|---|
| 58 | 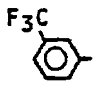 | H | H | $CH_3$ | $CH_3$ | Fp:82-87°C |
| 59 | 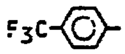 | H | H | $CH_3$ | $CH_3$ | $^1$H-NMR:1,2-1,9(6); 3,3-3,9(2);7,7s(4); |
| 60 | 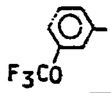 | H | H | $CH_3$ | $CH_3$ | Fp:90-93°C |
| 61 | 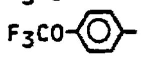 | H | H | $CH_3$ | $CH_3$ | Fp:75-80°C |
| 62 | 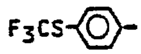 | H | H | $CH_3$ | $CH_3$ | Fp:60°C |
| 63 | 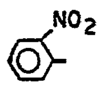 | H | H | $CH_3$ | $CH_3$ | $^1$H-NMR:1,2-1,8(6); 3,2-3,9(2);7,4-8,2(3); 8,4-8,6(1) |
| 64 | 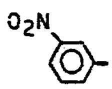 | H | H | $CH_3$ | $CH_3$ | Fp:158-160°C |
| 65 | 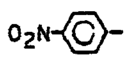 | H | H | $CH_3$ | $CH_3$ | Fp:65-70°C |
| 66 | 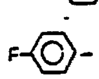 | H | H | $CH_3$ | $CH_3$ | Fp:90-95°C |
| 67 | 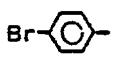 | H | H | $CH_3$ | $CH_3$ | Fp:77-79°C |
| 68 | 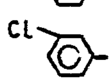 | H | H | $CH_3$ | $CH_3$ | $^1$H-NMR:1,1-1,9(6); 3,4-4,3(2);7,0-7.5(3); 7,55-7,8(1) |
| 69 | 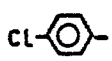 | H | H | $CH_3$ | $CH_3$ | Fp:97-98°C |
| 70 | 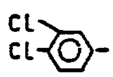 | H | H | $CH_3$ | $CH_3$ | Fp:88-90°C |
| 71 | 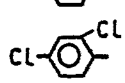 | H | H | $CH_3$ | $CH_3$ | Fp:105-108°C |
| 72 | 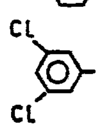 | H | H | $CH_3$ | $CH_3$ | Fp:113-115°C |
| 73 | 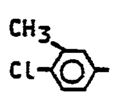 | H | H | $CH_3$ | $CH_3$ | $^1$H-NMR:1,0-1,8(6); 2,3s(3); 3,2-4,1(2); 7,1-7,5(3) |

0126380



| Bsp. Nr. | R | $R^1$ | $R^2$ | $R^3$ | $R^4$ | physikal. Eigenschaften |
|---|---|---|---|---|---|---|
| 74 | 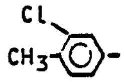 | H | H | $CH_3$ | $CH_3$ | Fp:102-104°C |
| 75 | 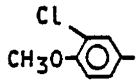 | H | H | $CH_3$ | $CH_3$ | Fp:93-95°C |
| 76 | 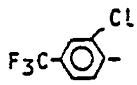 | H | H | $CH_3$ | $CH_3$ | Fp:78-80°C |
| 77 | 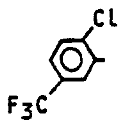 | H | H | $CH_3$ | $CH_3$ | $^1$H-NMR:1,3-1,8(6); 3,4-4,3(2);7,2-7,7(2); 8,5-8,7(1) |
| 78 | 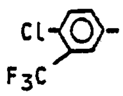 | H | H | $CH_3$ | $CH_3$ | Fp:104-106°C |
| 79 | 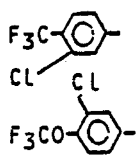 | H | H | $CH_3$ | $CH_3$ | $^1$H-NMR:1,1-1,9(6); 3,4-4,3(2); 7,5-7,7s (2); 7,75-8,0(1) |
| 80 | | H | H | $CH_3$ | $CH_3$ | Fp:79-86°C |
| 81 | 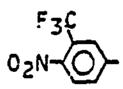 | H | H | $CH_3$ | $CH_3$ | Fp:79-80°C |
| 82 | 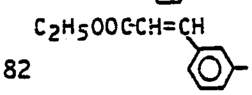 | H | H | $CH_3$ | $CH_3$ | $^1$H-NMR:1,0-2,0(9); 3,2-4,0(2);4,0-4,6q(2); 6,2-6,6(1);7,1-7,9(5) |
| 83 | 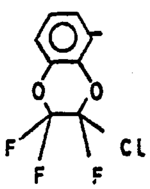 | H | H | $CH_3$ | $CH_3$ | $^1$H-NMR:1,0-1,9(6); 3,3-3,9(2);7,0-7,8(3) |
| 84 | 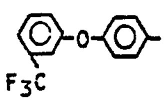 | H | H | $CH_3$ | $CH_3$ | $^1$H-NMR:1,2-1,9(6); 3,3-4,2(2);6,8-7,8(8) |
| 85 | 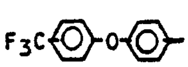 | H | H | $CH_3$ | $CH_3$ | $^1$H-NMR:1,0-1,6(6); 3,2-3,9(2);6,7-7,6(8); |
| 86 | 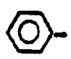 | H | $CH_3$ | $CH_3$ | $CH_3$ | Fp:61-62°C |

| Bsp. Nr. | R | $R^1$ | $R^2$ | $R^3$ | $R^4$ | physikal. Eigenschaften |
|---|---|---|---|---|---|---|
| 87 | 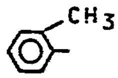 | H | $CH_3$ | $CH_3$ | $CH_3$ | Fp:69-72°C |
| 88 | 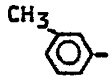 | H | $CH_3$ | $CH_3$ | $CH_3$ | $^1$H-NMR:1,2-1,8(9); 2,3s(3);3,5-4,1(1); 7,0-7,6(4) |
| 89 | 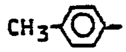 | H | $CH_3$ | $CH_3$ | $CH_3$ | $^1$H-NMR: 1,4-1,9(9); 2,35s(3); 3,5-4,2(1); 6,8-7,5(4) |
| 90 | 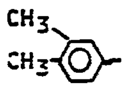 | H | $CH_3$ | $CH_3$ | $CH_3$ | $^1$H-NMR: 1,2-1,8(9); 2,2s(6); 3,5-4,2(1); 6,9-7,4(3) |
| 91 | 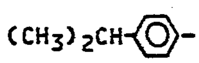 | H | $CH_3$ | $CH_3$ | $CH_3$ | $^1$H-NMR: 1,0-1,8(15); 2,5-3,0(1);3,5-4,0 (1); 6,9-7,5(4) |
| 92 | 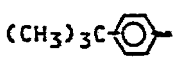 | H | $CH_3$ | $CH_3$ | $CH_3$ | $^1$H-NMR:1,0-1,8(18); 3,5-4,0(1);7,2-7,5(4); |
| 93 | 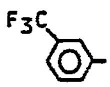 | H | $CH_3$ | $CH_3$ | $CH_3$ | $^1$H-NMR:1,0-1,8(9); 3,5-4,0(1);7,2-7,9(4); |
| 94 | 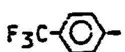 | H | $CH_3$ | $CH_3$ | $CH_3$ | $^1$H-NMR: 1,0-1,8(9); 3,5-4,1(1); 7,6(4) |
| 95 | 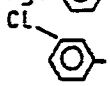 | H | $CH_3$ | $CH_3$ | $CH_3$ | Fp:74-79°C |
| 96 | 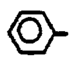 | H | H | $(CH_3)_2CH-$ | $(CH_3)_2CH-$ | Fp:59-65°C |
| 97 | 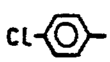 | H | H | $(CH_3)_2CH-$ | $(CH_3)_2CH-$ | Fp:102-120°C |
| 98 | 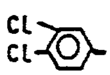 | H | H | $(CH_3)_2CH-$ | $(CH_3)_2CH-$ | Fp:91-98°C |
| 99 | 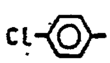 | H | H | $CH_3$ | $(CH_3)_2CH-$ | Fp:95-104°C |
| 100 | 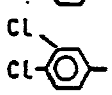 | H | H | $CH_3$ | $(CH_3)_2CH-$ | $^1$H-NMR:0,8-1,8(9); 2,0-2,9(1): 3,0-4,0(2); 7,4(2); 7,8(1); |
| 101 | 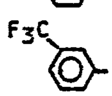 | H | H | $CH_3$ | $(CH_3)_2CH-$ | $^1$H-NMR:0,8-1,2(6); 1,2-3,7(3);2,0-2,9(1); 2,9-4,0(2);7,1-7,9(4) |

| Bsp. Nr. | R | $R^1$ | $R^2$ | $R^3$ | $R^4$ | physikal. Eigenschaften |
|---|---|---|---|---|---|---|
| 102 | 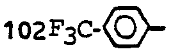 | H | $CH_3$ | H | $CH_3$ | Fp: 81-82°C |
| 103 | 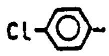 | H | $CH_3$ | $CH_3$ | $CH_3$ | $^1$H-NMR:1,2-1,9(9); 3,6-4,3(1); 7,2-7,7(4) |
| 104 | 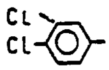 | H | $CH_3$ | $CH_3$ | $CH_3$ | $^1$H-NMR:1,2-1,9(9); 3,6-4,2(1);7,45(2); 7,85(1) |
| 105 | 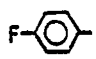 | H | $CH_3$ | $CH_3$ | $CH_3$ | $^1$H-NMR:1,3-1,9(9); 3,6-4,3(1);6,8-7,8(4); |
| 106 | 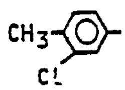 | H | $CH_3$ | $CH_3$ | $CH_3$ | $^1$H-NMR:1,3-1,9(9); 2,9(3); 3,4-4,3(1); 7,2-7,4(2); 7,75(1) |
| 107 | 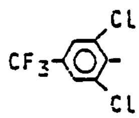 | H | $CH_3$ | $CH_3$ | $CH_3$ | $^1$H-NMR:1,1-1,9(9); 3,5-4,3(1); 7,7(2) |
| 108 | 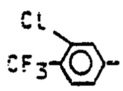 | H | $CH_3$ | $CH_3$ | $CH_3$ | $^1$H-NMR:1,3-1,9(9); 3,5-4,3(1);7,4-7,75(2); 7,75-8,0(1) |
| 109 | 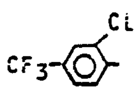 | H | $CH_3$ | $CH_3$ | $CH_3$ | $^1$H-NMR:1,3-1,9(9); 3,5-4,3(1);7,4-7,9(2); 8,4-8,7(1) |
| 110 | 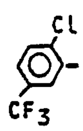 | H | $CH_3$ | $CH_3$ | $CH_3$ | Fp:91-92°C |
| 111 | 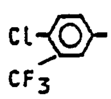 | H | $CH_3$ | $CH_3$ | $CH_3$ | $^1$H-NMR:1,3-1,9(9); 3,5-4,3(1);7,2-8,1(3) |
| 112 | 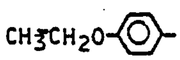 | H | $CH_3$ | $CH_3$ | $CH_3$ | Fp:68-69°C |
| 113 | 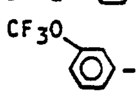 | H | $CH_3$ | $CH_3$ | $CH_3$ | $^1$H-NMR:1,3-1,9(9); 3,6-4,3(1);7,0-8,0(4); |
| 114 | 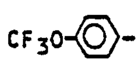 | H | $CH_3$ | $CH_3$ | $CH_3$ | $^1$H-NMR:1,3-1,9(9), 3,6-4,3(1);7,1-8,8(4) |

| Bsp. Nr. | R | $R^1$ | $R^2$ | $R^3$ | $R^4$ | physikal. Eigenschaften |
|---|---|---|---|---|---|---|
| 115 | 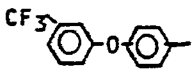 | H | $CH_3$ | $CH_3$ | $CH_3$ | $^1$H-NMR:1,3-1,9(9); 3,6-4,3(1);6,9-7,8(8) |
| 116 | 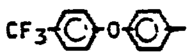 | H | $CH_3$ | $CH_3$ | $CH_3$ | $^1$H-NMR:1,3-1,9(9); 3,6-4,3(1);6,8-7,8(8) |
| 117 | 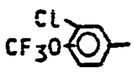 | H | $CH_3$ | $CH_3$ | $CH_3$ | $^1$H-NMR: 1,3-1,9(9); 3,6-4,3(1); 7,2-7,7(2); 7,9(1) |
| 118 | 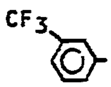 | H | H | H | $CH_3$ | Fp: 72-75°C |
| 119 | 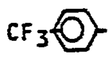 | H | H | H | $CH_3$ | Fp:75-78°C |
| 120 | 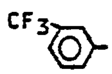 | H | $CH_3$ | H | $CH_3$ | Fp:61-63°C |
| 121 | $C_6H_5$ | H | H | H | $C_2H_5$ | $^1$H-NMR 1.0-1.5 m (3); 1.8 - 2.5 m (2); 3.0-4.0 m (3); 7.4-8.0 m (5) |
| 122 | $C_6H_5$ | H | H | H | $CH_3-(CH_2)_2-$ | $^1$H-NMR 0.8-1.0 t (3) 1.1-2.0 m (4); 3.0-3.5 m (3); 6.9 - 7.5 m (5) |
| 123 | $C_6H_5$ | H | H | H | $CH_3-(CH_2)_5-$ | $^1$H-NMR 0.7-1.0 t (3); 1.1 - 2.0 m (10); 3.0 - 3.6 m (3); 7.0 - 7.7 m (5) |
| 124 | 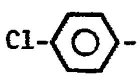 | H | H | H | $CH_3-(CH_2)_5-$ | Fp 82°C |
| 125 | 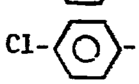 | H | H | H | $CH_3-(CH_2)_3-$ | Fp 71°- 73°C |
| 126 | 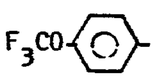 | H | H | H | $CH_3$ | Fp 62° - 63°C |
| 127 | 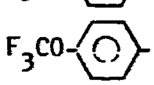 | H | H | H | $C_2H_5-$ | $^1$H-NMR 1.0 - 1.5 m (3); 1.5 - 2.5 m (2); 3.0 - 4.0 m (3); 7.3 - 8.3 m (4) |
| 128 | 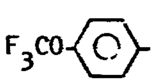 | H | H | H | $CH_3-(CH_2)_2-$ | NMR 0.8 - 1.3 t (3); 1.3 - 2.5 m (4); 3.1 - 4.0 m (3): 7.3 - 8.2 m (4) |
| 129 | 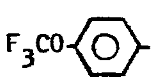 | H | $CH_3$ | H | $CH_3$ | $^1$H-NMR 0.7-1.3 m (6); 3.0- 3.7 m (3); 7.3 - 8.0 m (4) |

*Die $^1$H-NMR-Angaben geben die chemische Verschiebung in ppm an. Es handelt sich bei den Signalgruppen, wenn nicht anders angegeben, um Multipletts, die Zahl in Klammern gibt jeweils die Anzahl der zugeordneten Protonen an.

Anwendungsbeispiele:

In den folgenden Anwendungsbeispielen werden die nachstehend aufgeführten Verbindungen als Vergleichssubstanzen eingesetzt:

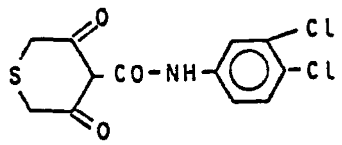

(A)

Tetrahydrothiopyran-3,5-dion-4-/N̄-(3,4-dichlorphenyl)/-carboxamid und

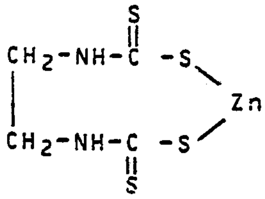

(B)

Zink-ethylen-1,2-bis-(dithiocarbamat)

Beispiel A

Fusarium nivale-Test (Roggen) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Roggen sät man mit 2 x 100 Korn 1 cm tief in eine Standarderde und kultiviert ihn im Gewächshaus bei einer Temperatur von ca. 10°C und einer relativen Luftfeuchtigkeit von ca. 95 % in Saatkästen, die täglich 15 Stunden dem Licht ausgesetzt werden.

Ca. 3 Wochen nach der Aussaat erfolgt die Auswertung der Pflanzen auf Symptome des Schneeschimmels.

Eine deutliche Überlegenheit in der Wirksamkeit gegenüber dem Stand der Technik zeigen in diesem Test z.B. die Verbindungen gemäß folgender Herstellungsbeispiele: 49, 99.

Beispiel B

Puccinia-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 58, 96, 101.

Beispiel C

Phytophthora-Test (Tomate) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 26, 28, 49, 53, 79 und 100.

Beispiel D

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 69 und 70.

Beispiel E

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 69, 99 und 100.

Beispiel F

Pyricularia-Test (Reis) / systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 46 und 70.

Patentansprüche

1. Substituierte Tetrahydrothiopyran-3,5-dion-4-carboxamide der allgemeinen Formel (I),

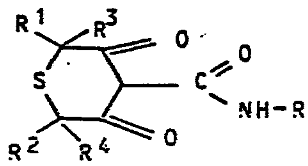

(I)

in welcher

R für Alkyl, für Cycloalkyl, für gegebenenfalls substituiertes Aralkyl oder für gegebenenfalls substituiertes Aryl steht,

$R^1$, $R^2$ und $R^3$ unabhängig voneinander für Wasserstoff oder für Alkyl stehen und

$R^4$ für Alkyl steht, ausgenommen die Verbindungen, in denen

$R^1$ und $R^3$ für Wasserstoff,

$R^2$ und $R^4$ für Methyl und

R für Alkyl, Phenyl oder durch Halogen und/oder Methyl substituiertes Phenyl steht oder in denen

$R^1$, $R^2$ und $R^3$ für Wasserstoff,

$R^4$ für Methyl und

R für Alkyl, Phenyl oder durch Halogen und/oder Methyl substituiertes Phenyl steht.

2. Substituierte Tetrahydrothiopyran-3,5-dion-4-carboxamide der allgemeinen Formel (I) gemäß Anspruch 1, in welcher

R für geradkettiges oder verzweigtes Alkyl mit bis zu 8 Kohlenstoffatomen, für Cycloalkyl mit 3 bis 7 Kohlenstoffatomen, für gegebenenfalls einfach oder mehrfach, gleich oder verschieden substituiertes Aralkyl mit 1 oder 2 Kohlenstoffatomen im Alkylteil und 6 bis 10 Kohlenstoffatomen im Arylteil, sowie für gegebenenfalls einfach oder mehrfach, gleich oder verschieden substituiertes Aryl mit 6 bis 10 Kohlenstoffatomen steht, wobei als Arylsubstituenten jeweils infrage kommen: Halogen, Cyano, Nitro, geradkettiges oder verzweigtes Alkyl, Alkoxy und Alkylthio mit jeweils bis zu 4 Kohlenstoffatomen, Halogenalkyl, Halogenalkoxy und Halogenalkylthio mit jeweils 1 oder 2 Kohlenstoffatomen und bis zu 5 gleichen oder verschiedenen Halogenatomen, gegebenenfalls durch Halogen oder niederes Alkyl einfach oder mehrfach, gleich oder verschieden substituiertes Dioxyalkylen mit 1 oder 2 Kohlenstoffatomen im Alkylenrest, gegebenenfalls einfach oder mehrfach, gleich oder verschieden durch Halogen,

niederes Alkyl und niederes Halogenalkyl substituiertes Phenoxy sowie Alkoxycarbonylalkyl und Alkoxycarbonylalkenyl mit jeweils 1 oder 2 Kohlenstoffatomen im Alkoxyteil und jeweils bis zu 2 Kohlenstoffatomen im Alkyl- bzw. Alkenylteil,

$R^1$, $R^2$ und $R^3$ unabhängig voneinander für Wasserstoff oder für geradkettiges oder verzweigtes Alkyl mit bis zu 6 Kohlenstoffatomen stehen und

$R^4$ für geradkettiges oder verzweigtes Alkyl mit 1 bis 6 Kohlenstoffatomen steht, ausgenommen die Verbindungen, in denen

$R^1$ und $R^3$ für Wasserstoff,

$R^2$ und $R^4$ für Methyl und

R für Alkyl, Phenyl oder durch Halogen und/oder Methyl substituiertes Phenyl steht oder

$R^1$, $R^2$ und $R^3$ für Wasserstoff

$R^4$ für Methyl und

R für Alkyl, Phenyl oder durch Halogen und/oder Methyl substituiertes Phenyl stehen.

3. Substituierte Tetrahydrothiopyran-3,5-dion-4-carboxamide der allgemeinen Formel (I) gemäß Anspruch 1, in welcher

R für geradkettiges oder verzweigtes Alkyl mit bis zu 6 Kohlenstoffatomen, für Cyclopentyl, für Cyclohexyl oder für gegebenenfalls ein- bis dreifach, gleich oder verschieden substituiertes Phenyl, Benzyl oder Naphthyl steht, wobei als Substituenten jeweils infrage kommen: Fluor, Chlor, Brom, Nitro, Cyano, Methyl, Ethyl, n- und i-Propyl, n-, i-, s- und t-Butyl, Methoxy, Ethoxy, Methylthio, Trifluormethyl, Trifluormethoxy, Trifluormethylthio, Dioxymethylen, Dioxyethylen, Dioxytrifluorchlorethylen, gegebenenfalls ein- bis dreifach, gleich oder verschieden durch Methyl, Chlor oder Trifluormethyl substituiertes Phenoxy, 2-Methoxycarbonylvinyl und 2-Ethoxycarbonylvinyl,

$R^1$, $R^2$ und $R^3$ unabhängig voneinander für Wasserstoff, Methyl, Ethyl, n- und i-Propyl sowie für n-, i-, s- und t-Butyl stehen und

$R^4$ für Methyl, Ethyl, n- und i-Propyl, n-, i-, s- und t-Butyl steht, ausgenommen die Verbindungen in denen

$R^1$ und $R^3$ für Wasserstoff,

$R^2$ und $R^4$ für Methyl und

R für Alkyl, Phenyl oder durch Halogen und/oder Methyl substituiertes Phenyl stehen oder in denen

$R^1$, $R^2$ und $R^3$ für Wasserstoff

$R^4$ für Methyl, und

R für Alkyl, Phenyl oder durch Halogen und/oder Methyl substituiertes Phenyl stehen.

4. Verfahren zur Herstellung der Tetrahydrothiopyran-3,5-dion-4-carboxamide der allgemeinen Formel (I)

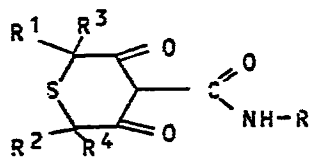

(I)

in welcher

R für Alkyl, für Cycloalkyl, für gegebenenfalls substituiertes Aralkyl oder für gegebenenfalls substituiertes Aryl steht,

$R^1$, $R^2$ und $R^3$ unabhängig voneinander für Wasserstoff oder für Alkyl stehen und

$R^4$ für Alkyl steht, ausgenommen die Verbindungen, in denen

$R^1$ und $R^3$ für Wasserstoff,

$R^2$ und $R^4$ für Methyl und

R für Alkyl, Phenyl oder durch Halogen und/oder Methyl substituiertes Phenyl steht oder in denen

$R^1$, $R^2$ und $R^3$ für Wasserstoff,

$R^4$ für Methyl und

R für Alkyl, Phenyl oder durch Halogen und/oder Methyl substituiertes Phenyl stehen, dadurch gekennzeichnet, daß man Tetrahydrothiopyran-3,5-dione der allgemeinen Formel (II)

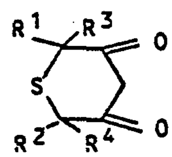

(II)

in welcher

$R^1$, $R^2$, $R^3$ und $R^4$ die oben angegebene Bedeutung haben,

mit Isocyanaten der allgemeinen Formel (III),

$$R - N = C = O \qquad (III)$$

in welcher

R die oben angegebene Bedeutung hat,

gegebenenfalls in Gegenwart eines Verdünnungsmittels und gegebenenfalls in Gegenwart einer Base umsetzt.

5. Schädlingsbekämpfungsmittel, gekennzeichnet durch einen Gehalt an mindestens einem substituierten Tetrahydrothiopyran-3,5-dion-4-carboxamid der Formel (I) gemäß Ansprüchen 1 und 4.

6. Verwendung von substituierten Tetrahydrothiopyran-3,5-dion-4-carboxamiden der Formel (I) gemäß Ansprüchen 1 und 4 zur Bekämpfung von Schädlingen.

7. Verfahren zur Bekämpfung von Schädlingen, dadurch gekennzeichnet, daß man substituierte Tetrahydrothiopyran-3,5-dion-4-carboxamide der Formel (I) gemäß Ansprüchen 1 und 4 auf Schädlinge und/oder ihren Lebensraum einwirken läßt.

8. Verfahren zur Herstellung von Schädlingsbekämpfungsmitteln, dadurch gekennzeichnet, daß man substituierte Tetrahydrothiopyran-3,5-dion-4-carboxamide der Formel (I) gemäß Ansprüchen 1 und 4 mit Streckmitteln und/oder oberflächenaktiven Mitteln vermischt.

9. Tetrahydrothiopyran-3,5-dione der allgemeinen Formel (II)

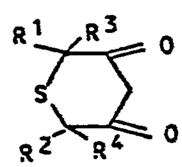

(II)

in welcher

$R^1$, $R^2$ und $R^3$ unabhängig voneinander für Wasserstoff oder Alkyl stehen und

$R^4$ für Alkyl steht, ausgenommen die Verbindungen, in denen

$R^1$, $R^2$ und $R^3$ für Wasserstoff und

$R^4$ für Methyl oder i-Propyl steht oder in denen

$R^1$ und $R^3$ für Wasserstoff und

$R^2$ und $R^4$ für Methyl stehen.

Europäisches Patentamt

# EUROPÄISCHER RECHERCHENBERICHT

Nummer der Anmeldung
EP 84 10 5236

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| D,A | CHEMICAL ABSTRACTS, Band 87, 1977, Seiten 580-581, Nr. 84822b, Columbus, Ohio, US; & JP - A - 77 46 078 (NIPPON SODA CO., LTD.) 12.04.1977 * Zusammenfassung * --- | 1,2,4-9 | C 07 D 335/02<br>A 01 N 43/18 |
| D,A | US-A-3 833 610 (G. HARDTMANN) * Spalten 1-6 * ----- | 1,2,4-9 | |
| | | | RECHERCHIERTE SACHGEBIETE (Int. Cl. 3) |
| | | | C 07 D 335/00 |

Der vorliegende Recherchenbericht wurde für alle Patentansprüche erstellt.

| Recherchenort | Abschlußdatum der Recherche | Prüfer |
|---|---|---|
| DEN HAAG | 23-08-1984 | FRANCOIS J.C.L. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82